# EUROPEAN PATENT SPECIFICATION

(11) **EP 2 736 318 B1**
(45) Date of publication and mention of the grant of the patent: **17.08.2016**
(21) Application number: 12746194.5
(22) Date of filing: 27.07.2012
(51) Int. Cl.: A01G 7/00

(54) **PLANT GROWTH KINETICS CAPTURED BY MOTION TRACKING**
DURCH BEWEGUNGSVERFOLGUNG AUFGENOMMENE PFLANZENWACHSTUMSKINETIK
CINÉTIQUE DE LA CROISSANCE VÉGÉTALE CAPTURÉE PAR SUIVI DE MOUVEMENTS

(30) Priority: 27.07.2011 US 201161512291 P
(43) Date of publication of application: 04.06.2014
(73) Proprietor: Dow AgroSciences LLC, Indianapolis, IN 46268 (US)
(72) Inventor: SETLUR, Pradeep, Carmel, IN 46033 (US); PAI, Reetal, Carmel, IN 46033 (US); GOLGOTIU, Kirsti Alise, Oregon City, OR 97045 (US); BEATTY, Douglas, Newberg, OR 97132 (US)
(74) Representative: Beck Greener
(86) International application number: PCT/US2012/048455
(87) International publication number: WO 2013/016603

(56) References cited:
- US-A1- 2007 289 207
- JAMES W. HART ET AL: "Analysis of growth during phototropic curvature of cress hypocotyls", PLANT, CELL & ENVIRONMENT, [Online] vol. 5, no. 5, 1 October 1982 (1982-10-01) , pages 361-366, XP55041107, ISSN: 0140-7791, DOI: 10.1111/j.1365-3040.1982.tb00935.x Retrieved from the Internet: URL:http://onlinelibrary.wiley.com/doi/10. 1111/j.1365-3040.1982.tb00935.x/pdf> [retrieved on 2012-10-15] cited in the application
- I. R. MACDONALD ET AL: "Cyclamen coiling--the migration of a growth response.", PLANT, CELL & ENVIRONMENT, [Online] vol. 10, no. 7, 1 September 1987 (1987-09-01), pages 613-617, XP55041108, ISSN: 0140-7791, DOI: 10.1111/1365-3040.ep11604129 Retrieved from the Internet: URL:http://onlinelibrary.wiley.com/doi/10. 1111/j.1365-3040.1982.tb00935.x/pdf> [retrieved on 2012-10-15] cited in the application
- JULIEN DIENER: "Acquisition et génération du mouvement des plantes", Institut Polytechnique de Grenoble , 4 December 2009 (2009-12-04), XP002685283, Grenoble, FR Retrieved from the Internet: URL:http://tel.archives-ouvertes.fr/docs/0 0/43/87/78/PDF/thesisDiener.pdf [retrieved on 2012-10-15]
- M. J. JAFFE ET AL: "Computer-Assisted Image Analysis of Plant Growth, Thigmomorphogenesis, and Gravitropism", PLANT PHYSIOLOGY, [Online] vol. 77, no. 3, 1 March 1985 (1985-03-01), pages 722-730, XP55041153, ISSN: 0032-0889, DOI: 10.1104/pp.77.3.722 Retrieved from the Internet: URL:http://www.plantphysiol.org//content/7 7/3/722.full.pdf> [retrieved on 2012-10-15] cited in the application

## Description

### FIELD OF THE DISCLOSURE

The present disclosure relates to use of motion sensing and tracking equipment to image, monitor, track, and/or determine a parameter of plant growth kinetics (*e.g*., plant leaf elongation and height growth rate). An apparatus of the disclosure and its associated methods may be used to screen plants for the presence of one or more agronomic trait(s), and/or to study the growth kinetics of particular plants and cultivars, for example, in a high-throughput platform.

### BACKGROUND

Biologists have long desired to accurately measure and predict plant growth. Conventional growth-studies using a ruler have provided valuable information about cumulative plant growth over long time periods. Depending on the particular plant, measurements of growth parameters are usually taken at a daily interval to obtain significant results. As a consequence of this relatively long interval period, detailed information about the daily growth pattern and possible circadian rhythms in growth rate are lost. Furthermore, repeated and transient physical contact with the plants during measurement may lead to thigmomorphogenic phenomena, which result in an altered growth behavior. Jaffe (1976) Z. Pflanzenphysiol. 78:24-32. When physically measuring plant growth, the more frequently growth is measured, the more frequently the plant must be physically contacted and thereby have its growth rate subjected to thigmomorphogenic alteration. Thus, these problems are related.

Plants are capable of responding rapidly (*e.g.*, with responses that cannot be detected by daily measurement) to changes in environmental factors, such as irradiance, light quality and temperature. For example, several short-term growth studies have revealed so-called "growth-rate transients." Such transients, while not necessarily making any significant contribution to total plant elongation, may provide information about the existing differences in kinetics of growth responses. Cosgrove (1981) Plant Physiol. 67:584-90; Gaba and Black (1983) Photochem. Photobiol. 38:473-6; Kristie and Jolliffe (1986) Can. J. Botany 64:2399-405; and Prat and Parésys (1995) Plant Physiol. Biochem. 33:709-16. In order to detect rapid growth responses, such as growth-rate transients, appropriate measuring methods must have both a sampling frequency high enough to capture the phenomenon and high sensitivity, since such growth-rate changes may be less than a few micrometers per second.

Methods for the continuous measurement of plant growth have been continuously developing since the use of autographic auxanometers in the late 19^{th} century to monitor diurnal fluctuations of growth in stem length. *See* Anderson and Kerr (1943) Plant Physiol. 18:261-9; and Wilson (1948) Plant Physiol. 23:156-7. To obtain high-resolution measurements of elongation changes, new methods were developed to enable accurate continuous registration of growth of intact plants. *See*, *e.g.*, Meijer (1968) Acta Bot. Neerl. 17:9-14 (linear variable differential transformers). Since then, displacement transducers have frequently been applied to study rapid growth responses to light and other environmental conditions. Penny et al. (1974) Can. J. Botany 52:959-69; Cosgrove (1981), *supra*; Van Volkenburgh et al. (1983) Ann. Bot. 51:669-72; Lecharny et al. (1985) Plant Physiol. 79:625-9; Child and Smith (1987) Planta 172:219-29; Shinkle and Jones (1988) Plant Physiol. 86:960-6; Prat and Parésys (1989) Plant Physiol. Biochem. 27:955-62; Bertram and Karlsen (1994) Sci. Hort. 58:139-50; and Ruiz Fernandez and Wagner (1994) J. Plant Physiol. 144:362-9.

Optical growth analysis methods, such as, for example, the interferometric measurement technique (Fox and Puffer (1976) Nature 261:488-90; and Jiang and Staude (1989) J. Exp. Bot. 40:1169-73); time-lapse photography (Hart et al. (1982) Plant Cell Environ. 5:361-6; and Baskin et al. (1985) Plant Cell Environ. 8:595-603); and video registration (Jaffe et al. (1985) Plant Physiol. 77:722-30; MacDonald et al. (1987) Plant Cell Environ. 10:613-7; and Popescu et al. (1989) Photochem. Photobiol. 50:701-5), have alternatively been used. These methods have the advantage of not requiring physical contact with the plant.

### BRIEF SUMMARY OF THE DISCLOSURE

Described herein are apparati and methods related to application of motion sensing and tracking technology in an apparatus adapted for imaging, monitoring, tracking, and/or determining a parameter of plant growth kinetics (*e.g*., plant leaf elongation and height growth rates). Some embodiments provide advantageous and alternative compositions and methods to conventional systems for the capture of plant kinetics, including conventional optical growth analysis methods. Conventional systems for the capture of plant kinetics typically involve either manual capture by measuring leaf length and plant height over time, or digital image capture by processing serial images using colorimetric or manual image processing. Both of these procedures are costly and labor intensive. Some embodiments of the invention provide an automated platform that may greatly enhance the throughput for plant screening applications of agronomic traits (*e.g*., drought tolerance, NUE, heat tolerance, salt tolerance, etc.) by reducing the time required for data acquisition and/or analysis. In particular embodiments, the use of reflective markers in conjunction with one or more cameras adapted to enhance imaging of the markers to measure plant growth may provide an efficient and cost-effective approach for measuring plant growth kinetics in the agricultural research field.

Some embodiments may include a plant sample growth motion tracking and imaging station. In particular embodiments, a plant sample growth motion tracking and imaging station may comprise a sample volume adapted to contain at least one plant sample (*e.g*., a plant, plant part, or plant tissue) having at least one marker(s) defining physical points on the plant sample, a plurality of marker sensors arranged at the periphery of the sample volume, and a motion capture processor coupled to the plurality of marker sensors to receive motion capture data from the plurality of marker sensors, and produce a digital representation reflecting growth of the plant sample. In these and further embodiments, a plant sample growth motion tracking and imaging station may produce a digital representation that comprises spatial plant growth information in three dimensions and temporal plant growth information.

Some embodiments may include a plant sample growth motion tracking and imaging station comprising a sample volume adapted to contain more than one plant sample at the same time. Some embodiments may include a motion capture processor that is capable of performing image capture and image processing functions. In some embodiments, a plant sample growth motion tracking and imaging station further comprises a workstation and a data storage device, wherein the workstation and data storage device are coupled to the motion capture processor.

In particular embodiments exemplified herein, a plant sample growth motion tracking and imaging station comprises marker sensors that may be cameras. Plant samples that are introduced into such a plant sample growth motion tracking and imaging station may have one or more markers that either emit light that is detected by the cameras, or reflect light emitted elsewhere to the cameras for detection. In these and further embodiments, a plant sample growth motion tracking and imaging station may further comprise a means for moving the sample volume without moving the marker sensors in relation to the sample volume. An example of such a means for moving the sample volume without moving the marker sensors in relation to the sample volume is a moveable overhead gantry comprising at least one of the plurality of marker sensors.

Also described herein are methods for monitoring the growth kinetics of a plant sample. Such methods may comprise in some embodiments: defining a sample volume adapted to contain at least one plant sample having at least one marker(s) defining physical points on the plant sample; arranging a plurality of marker sensors at the periphery of the sample volume; acquiring motion capture data utilizing the plurality of marker sensors; receiving motion capture data from the plurality of marker sensors; and producing a digital representation reflecting growth of the plant sample. In particular embodiments, a digital representation comprises spatial information in three dimensions and temporal information.

Also described are automated methods for monitoring the growth kinetics of a plant sample. Such methods may comprise in some embodiments: defining a sample volume adapted to contain at least one plant sample having at least one marker(s) defining physical points on the plant sample; attaching a plurality of marker sensors to a means for moving the sample volume without moving the marker sensors in relation to the sample volume; arranging the plurality of marker sensors at the periphery of the sample volume; introducing into the sample volume a plant sample having at least one marker(s) defining physical points on the plant sample; acquiring motion capture data utilizing the plurality of marker sensors; repeating the sample introduction and the data acquisition with a next plant sample; and receiving motion capture data from the plurality of marker sensors and producing a digital representation reflecting growth of the plant sample

These manual and automated methods may be used in some embodiments for screening a plant sample for a growth trait of interest. In these and other embodiments, the method may comprise: defining a sample volume adapted to contain at least one plant sample having at least one marker(s) defining physical points on the plant sample; arranging a plurality of marker sensors at the periphery of the sample volume; introducing into the sample volume a plant sample having at least one marker(s) defining physical points on the plant sample; acquiring motion capture data utilizing the plurality of marker sensors; receiving motion capture data from the plurality of marker sensors and producing a digital representation reflecting growth of the plant sample; and analyzing the digital reflection to determine whether the plant sample comprises the growth trait of interest.

The use of screening methods according to some embodiments may greatly facilitate the identification and study of plant growth phenotypes, and recognition of plants and plant cultivars having such phenotypes, even when such phenotypes are difficult or impossible to recognize by conventional techniques and methods. For example, small changes in plant parts or tissues that are representative of plant growth that occurs transiently or dynamically may be perceived, measured, and modeled according to particular embodiments of the present invention, even when these transient or dynamic processes are invisible to the naked eye or to measurement techniques having a low sampling frequency.

The foregoing and other features will become more apparent from the following detailed description of several embodiments, which proceeds with reference to the accompanying figures.

### BRIEF DESCRIPTION OF THE FIGURES

**FIG. 1** includes a block diagram illustrating an apparatus in accordance with some embodiments.
**FIG. 2** includes a top view of a sample volume with a plurality of cameras adapted to enhance imaging of markers to image plant growth arranged within the sample volume.
**FIG. 3** includes a side view of the sample volume with a plurality of cameras adapted to enhance imaging of markers to image plant growth arranged within the sample volume.
**FIG. 4** includes a top view of the sample volume illustrating an exemplary arrangement of the plurality of cameras with respect to a quadrant of the sample volume.
**FIG. 5** includes a top view of the sample volume illustrating an exemplary arrangement of the plurality of cameras with respect to comers of the sample volume.
**FIG. 6a** includes an illustration of an exemplary apparatus according to some embodiments, including a motion tracking imaging station, a camera array connected to an automated overhead gantry, attached equipment, and equipment connections. FIG. 6b illustrates another example of such an apparatus, including a high-throughput gravimetric automation platform.
**FIG. 7** includes formulas useful for three-dimensional coordinate distance calculations and kinematics analysis.
**FIG. 8** includes screenshot pictures of the tracking tools image capturing screenshots, and the marker set that is detected in the field of view in relation to the set surface level plane.
**FIG. 9** includes the daily change in three-dimensional position in space from the initial location to the final location.
**FIG. 10** includes cumulative displacement (values in meters) of markers attached to three different leaves over time.
**FIG. 11** includes screenshot pictures of tracking tools image capture over the course of 12 days.
**FIG. 12** includes daily leaf displacement values for multiple plants measured using the motion tracking tools.

### DETAILED DESCRIPTION

### I. Overview of several embodiments

Described herein is specifically-adapted motion tracking equipment, and methods of their use, for imaging, monitoring, tracking, and/or determining a parameter of plant growth kinetics. In some embodiments, markers are used in conjunction with a set of marker sensors adapted to enhance detection of the markers to determine the position of a fixed location on a plant, plant part, or plant tissue over time. Parameters of plant growth kinetics may be calculated and extrapolated from such time-dependent position data. In particular embodiments, a composition, method, and/or apparatus for capturing plant growth kinetics by motion tracking may be used to compare the growth of individual plants under the same or different environmental conditions, thereby providing a relatively inexpensive, rapid, and/or high-throughput system for screening plants for specific agronomic traits.

The broad utility of the presently disclosed system has been exemplified and validated by detecting differences in growth rates between well-watered and drought-treated plants that may have avoided detection by conventional tools and methods. Thus, some embodiments include methods of screening plants for a trait or phenotype that has an effect on the growth of a plant. Such traits and phenotypes include, for example and without limitation, drought tolerance, NUE, heat tolerance, and salt tolerance.

In certain exemplary embodiments, a reflective marker material may be applied to the surface of a plant sample (*e.g.*, a plant, plant part, or plant tissue). The marker may be applied without damaging the plant sample, and the marker may remain visible to at least one marker sensor under greenhouse conditions for the length of a planned growth experiment. The position of the marker may be determined, for example and without limitation, by using a standard digital camera flash under completely dark conditions. The position of the marker material over time may be tracked by image analysis on a two-dimensional plane, and/or in a three-dimensional volume.

In these and other embodiments, the motion sensing and tracking equipment and apparatus may be automated, and such may facilitate high-throughput data acquisition and analysis with minimal physical interaction between the practitioner and the sample plants.

### II. Abbreviations

- ATSC: digital television transmission standards developed by the Advanced Television Systems Committee
- CG: computer graphics
- IR: infrared
- LED: light-emitting diode
- NUE: nitrogen use efficiency
- NTSC: analog television transmission standards developed by the National Television Systems Committee
- V4: fourth leaf stage
- V6: sixth leaf stage

### III. Terms

Frame: As used herein, the term "frame" refers to a period of time, or a collection of different periods of time, at which a position of a marker being captured by motion tracking is calculated. For example, in some embodiments, a frame may be a period beginning at the time a marker position is calculated, and ending at the time a next marker position is calculated. In these and other embodiments, a frame may include additional marker position calculations between the beginning- and end-point calculations. For example, a frame may be the period of time during which a complete motion tracking study or experiment is conducted. In some examples, this complete frame may be alternatively referred to as the "complete sampling period."

Markers: As used herein, the term "marker" refers to a means for marking a specific location on a three-dimensional object in space. The position of a marker in space at a particular time may be determined by a marker sensor (*e.g.*, a camera). Markers include, but are not limited to, passive markers, active markers, reflective markers, retro-reflective markers, LED markers, photosensitive markers, radio-transmitter markers, acoustic markers, inertial markers, magnetic markers, and combinations of any of the foregoing. In some embodiments, a reflective marker may be illuminated by light of a particular wavelength or wavelength range (*e.g.*, IR) emitted from an LED mounted around or on a camera lens (*e.g*., with an IR pass filter placed over the camera lens), and the reflective marker may reflect the light to the camera lens, which may detect the reflected light. In particular embodiments, highly-reflective bead markers are used in conjunction with one or more cameras adapted to enhance imaging of the markers to image plant growth. A camera may be adapted to enhance imaging of a marker, for example, through the use of a filter.

Centroid: The centroid of a marker is a position estimated within a captured two-dimensional image. In embodiments utilizing a camera as a marker sensor, the grayscale value of each pixel acquired by the camera may be used to estimate the position of the centroid of a particular marker with sub-pixel accuracy, by finding the centroid of a fitted Gaussian function of the marker position.

Motion tracking: As used herein, "motion tracking" encompasses a large and varying collection of technologies for recording and/or calculating the displacement of one or more locations on a three-dimensional object over time in a sample volume (*e.g.*, image-based systems, such as optical systems). Image-based systems determine the position in three-dimensions of predetermined points (*e.g*., marker locations) on a three-dimensional object by using multiple cameras to each record the position of the points in a two-dimensional image. Displacement is recorded/calculated through the capture of multiple two-dimensional images of the sample volume, including the object, corresponding to sequential frames. Stereometric techniques correlate predetermined points on an object in each image, and use this correlative information with a known relationship between each of the images and camera parameters to calculate point position.

Optical motion tracking systems ("optical systems") utilize data captured from optical marker sensors to triangulate the three-dimensional position of a marker in the field of view of one or more cameras ("capture area") calibrated to provide overlapping two-dimensional image projections. Tracking a large number of objects in the sample volume, or expanding the capture area, may be accomplished by the addition of more cameras. In some embodiments, an optical system produces data with three degrees of freedom for each marker, and rotational information may be inferred from the relative orientation of three or more markers. Early in the evolution of optical motion tracking systems, such systems could only track about a dozen markers in one sample volume. However, more recent optical systems can track more than 100 markers simultaneously in real-time.

Optical motion tracking systems may use any of many lighting systems known in the art. For example, "structured light systems" use lasers or beamed light to create a plane of light that is swept across the sample volume. Optical systems based on pulsed-LEDs measure light emitted by one or more LEDs placed at predetermined points on the object. Optical systems based on reflective markers measure light reflected by one or more marker(s), each placed at a predetermined point on the object. Optical systems potentially suffer from occlusion (line of sight) problems whenever a required light path (such as the path from a reflective marker to a photosensitive marker sensor) is blocked. Interference from other light sources or reflections may also be a problem in embodiments where such other light sources or reflections are detectable in the sample volume. Such interference may result in "ghost markers."

Optical systems based on camera motion tracking of an object without the use of markers is also possible ("markerless motion tracking"), but is generally less accurate than marker-based systems. Nonetheless, markerless motion tracking may be desirable in some embodiments, for example, where physical contact of an object with a marker is desirably completely avoided. Markerless motion tracking is described in more detail below.

"Active optical systems" triangulate marker positions by illuminating one light source marker (*e.g.*, a LED located in or on a marker) at a time very quickly, or multiple light source markers at a time, and using software to identify the markers by their relative positions. In an active optical system, rather than reflecting light that is generated externally, the markers themselves emit their own light. By providing 1/4 the power at two times the distance (Inverse Square law), active optical systems may increase the distances and sample volume for motion capture. Power to each marker may be provided sequentially in phase with the capture system, thereby providing a unique identification of each marker for a given capture frame. The ability to identify each marker in this manner may be useful in real-time applications. An alternative method of identifying markers in an active optical system is to do it algorithmically.

Active marker systems may be further refined by illuminating one marker at a time (*i.e*., "strobing"), or tracking multiple markers over time and modulating the amplitude or pulse width to uniquely-identify particular markers. Unique marker identifications may reduce the time required for data processing, for example, by eliminating marker swapping and providing much cleaner data than other technologies. LEDs with onboard processing and radio synchronization allow motion capture outdoors in direct sunlight, while capturing at least 480 frames per second through the use of a high-speed electronic shutter. Computer processing of modulated markers allows less "hand cleanup" or filtered results, and thus may lead to lower operational costs.

"Passive optical system" use markers that are constructed from, or coated with, a reflective material to reflect light emitted elsewhere to the cameras lens. In particular embodiments, a reflective marker sensor's threshold may be adjusted, such that only the reflective markers will be sampled, for example, to the exclusion of light reflected by natural plant material. Unlike active systems and magnetic systems, passive systems do not require that wires or electronic equipment be attached to the tracked object. Instead, small objects (*e.g*., rubber balls or beads) may be attached only to a location on the moving object. These small objects may be attached by any suitable attachment means (*e.g*., tape, Velcro, ties, etc.). The small object may themselves be reflective, or a non-reflective object may be attached to the moving object with a reflective attachment means. Reflective markers for use in a passive optical system may be smaller and lighter than active system markers, and thus their use may result in less of a physical disturbance to a growing plant. Accordingly, in some embodiments, passive optical systems are preferred.

"Non-traditional motion tracking," wherein specially-built multi-LED IR projectors optically encode the sample volume, and photosensitive markers to decode the optical signals, may also be used in some embodiments. An example of one such non-traditional system is Prakash™, which uses multi-LED high speed projectors. By attaching "decoding markers" (so-called, because these markers decode signals, instead of transmitting a signal that is decoded by separate equipment) with photo sensors to locations on a plant in the sample volume, the decoding markers can compute, not only their own positions in the sample volume over time, but also their orientations, incident illuminations, and reflectances. Such decoding markers may work in natural lighting conditions, and may be attached to a location on a plant by any suitable attachment means. Non-traditional motion tracking systems may support an unlimited number of markers in a sample volume, with each marker uniquely-identified to eliminate marker reacquisition issues. Since such a system may eliminate the need for a high speed camera, and a corresponding high-speed image stream, it requires significantly lower data bandwidth. As previously noted, decoding markers may also provide incident illumination data, which may be used to monitor changes in the light experienced by the plant at the location of the marker over time.

Markerless motion capture: There has recently been rapid development in the area of "markerless motion capture." Current markerless systems are largely based on computer vision techniques of pattern recognition, and they often require considerable computational resources. Special computer algorithms have been designed to allow the system to analyze multiple streams of optical input, identify the forms of objects contained in the sample volume, and computationally reduce the objects into constituent parts for tracking. Examples of methods for markerless motion tracking are described, for example, in U.S. Patent 7,257,237.

Trait: As used herein, the term "trait" refers to a measurable characteristic of an individual. Certain traits may be useful in grouping or typing several individuals into a single cohort. The terms "trait" and "phenotype" are used interchangeably herein. Of particular interest in some embodiments of the invention are traits relating to plant growth and/or morphology.

Some embodiments include one or more "agronomic trait(s)." As used herein, the term "agronomic trait" may refer to traits such as, for example and without limitation, increased or altered growth characteristics, stress tolerance (*e.g*., drought, NUE, heat, salt, etc.), disease and insect resistance, modified seed oil composition, modified seed protein, and expression of one or more transgene(s) in a transgenic organism. Some examples include agronomic traits that result in increased or decreased plant growth in a particular environmental condition or set of conditions.

### IV. Capture of plant growth kinetics by motion tracking and imaging

Methods and compositions described herein employ motion sensing and tracking to address problems in plant growth, morphology, and physiology. In some embodiments, motion sensing and tracking equipment, software, and techniques may be utilized to accurately quantify parameters of plant growth; *e.g*., plant leaf elongation, and plant height. In particular embodiments, such parameters may be quantified in real-time, and optionally in a fully-automated manner.

### Image Acquisition

Typically, motion tracking utilizes one or more markers in a three-dimensional space (referred to in some places herein as the "sample volume"), and motion tracking equipment is configured to precisely locate the marker(s) in that space. Motion tracking equipment may be used to sample the location of the marker(s) at a high frequency. When a series of such samples are analyzed, the movement of the marker(s) over the time of the experiment may be reconstructed. In optical systems, the selection of the time interval for imaging depends, for example, on the speed required to capture the displacement of the object as it occurs. Depending on the light source used, the selection of the interval may also depend on the requirements of the system (*e.g*., to keep laser light low due to its damaging effect on plants). The size of the marker(s) used may impact the sensitivity of the marker location sampling. However, by locating the marker's centroid, displacements smaller than the diameter or width of the marker may be measured.

Occlusion occurs in an optical motion tracking system when a required light path between a marker and a marker sensor is blocked, for example, by a plant part that moves between the marker and the sensor during plant growth. Occlusion may be overcome by the use of more cameras. Further, manual post-processing may be used to recover trajectories when a marker is lost from view. The selection of a particular camera for use in some embodiments is within the discretion of the practitioner, and may involve consideration of many variables, including for example and without limitation, compatibility with other equipment, cost, field of view, space of movement, image acquisition rate, and resolution. For example, a particular camera generally can provide greater displacement resolution if focused on a smaller field of view, but this limits the size of the displacements that may be tracked. In view of such limitations of particular systems, post-processing procedures to analyze, process, and clean up data may be utilized with such systems before they are applied.

In some embodiments utilizing an optical system, an object with markers attached at known positions may be used to calibrate the cameras and obtain their positions, and the lens distortion of each camera may be measured. If two calibrated cameras can locate a particular marker in each of their two-dimensional imaging fields, a three-dimensional position of the marker may be obtained. In some embodiments, an optical system may comprise about two, three, or more cameras. For example, in particular embodiments, an optical system may comprise between about 2 and about 25 cameras (*e.g*., 1, 2, 3, 4, 5, 6, 7, 8, 9, 10, 11, 12, 13, 14, 15, 16, 17, 18, 19, 20, 21, 22, 23, 24, 25, 26, and 27 cameras). Systems of more than 100 cameras exist to reduce marker swap. The addition of more cameras may be required in some examples to provide for full coverage of the sample volume and tracking of motion in multiple plants. "Marker swapping" may occur in embodiments utilizing markers that are identical with respect to the marker sensors. Constraint software that reduces marker swapping is commercially available from several sources.

Optical motion tracking systems may, in some embodiments, capture large numbers of markers at frame rates as high as, for example, 2000 Hz. The frame rate available for a given is a balance between resolution and speed. NTSC video typically provides a sampling rate of approximately 30 Hz. Those of skill in the art will appreciate that most plant growth processes occur at rates that are slow enough that they may be successfully tracked at much lower sampling rates. Thus, NTSC or ATSC video will generally be sufficient for most applications and in most embodiments.

In embodiments of the present invention, any motion tracking system may be used, and the selection of a particular system is within the discretion of the practitioner, in view of the plant growth phenomenon to be tracked, and other design considerations. For example and without limitation, image-based systems, optical systems, active systems, passive systems, non-traditional systems, markerless optical systems, magnetic systems, mechanical systems, and radiofrequency systems may be used in particular embodiments. Optical motion tracking systems that may be useful in particular embodiments include, for example and without limitation, an MX™ camera system (Vicon Peaks Inc.), an OptiTrack™ (Natural Point Inc.), and a Hawk Digital System™ (Motion Analysis Corp.).

### Image Processing

In some embodiments, image processing and analysis may be used in a motion tracking system, *inter alia*, to determine the shape and size of plants and plant tissues present in a sample volume, and to determine the spatiotemporal relationship between discrete locations on such plants and plant tissues. Image processing may produce data for the design, validation, and optimization of plant growth process models. Such data, which embodiments of the invention allow to be acquired with unprecedented accuracy for macroscopic plant growth processes, allows the assembly of realistic computational representations that depict the geometry of growing plants, plant parts, and plant tissues, and networks of connections there between, that may form the basis for mechanical models. In general, image processing may provide a detailed geometric description of growing plants, plant parts, and plant tissues, necessary for the resizing and reshaping of plants as they grow, which can form the basis for growth models. Some exemplary aspects of image processing may include visualization of spatiotemporal data; segmentation (extraction of features from images); computation of growth; and/or creating a realistic geometry for a model.

Visualization of spatiotemporal data: Visualizing a spatiotemporal dataset produced by an optical motion tracking system generally requires specialized software. Image analysis packages with volume rendering capabilities allow the creation of three-dimensional visualizations that can be rotated freely and cropped. Contrast enhancement, reduction of noise, deblurring, and similar procedures may also enhance images in preparation for segmentation.

Segmentation: The computation of shape and size may be determined as the output of an image-processing pipeline including segmentation. Image segmentation is the process of partitioning an image into distinct regions each representing a single homogeneous object. Gonzalez and Woods (2008) Digital Image Processing. Upper Saddle River, N.J.: Prentice Hall. With regard to the quantification of morphology, segmentation generates digital masks from which volume, area, length, and shape properties may be calculated for each segmented object (*e.g.*, a plant part or tissue). Segmentation may also be used to compute the connectivity (topology) between objects, by generating digital masks for each object, and subsequently finding their neighbors.

Translating imaging to realistic geometry for models: The reconstruction of the spatiotemporal relationship between plant, plant part, and/or plant tissue geometry and topology from two-dimensional images allows the construction of finite element meshes needed for plant growth process simulations.

### V. Plant growth motion tracking and imaging station

In accordance with the foregoing, some embodiments include an apparatus and method for capturing plant growth kinetics data, comprising a sample volume adapted to contain at least one plant sample (*e.g*., a plant, plant part, or plant tissue) having positional markers defining one or more physical locations on the sample. The sample volume may have any desired geometric shape, for example and without limitation, oval, round, rectangular, square, and polygonal. A plurality of marker sensors (*e.g*., cameras) may be arranged around, along, or within a periphery of the sample volume. Marker sensors may be arranged such that the positional markers while the plant is growing are within the field of view of at least one of the marker sensors at substantially all times during a data acquisition period. A motion capture processor may be coupled to the marker sensors to produce a digital model reflecting the movement of the markers during a data acquisition period (*i.e.*, the growth of the plant sample during this period).

In some embodiments, the sample volume may comprise a planar area subdivided into a plurality of quadrants. The quadrants may each further comprise a plurality of edges coincident with the periphery of the sample volume. The plurality of marker sensors may comprise a first portion of the plurality of marker sensors that are disposed at a first height above the lowest point of the sample volume, and at least a second portion of the plurality of marker sensors that are disposed at a second height above the lowest point of the sample volume that is greater than the first height. In some embodiments, the sample volume may also comprise at least one light source (*e.g*., a polarized light source, and a filtered light source) oriented to illuminate all or a portion of the sample volume. For example, a light source may be oriented to illuminate all or a portion of a plant growing within the sample volume. In particular examples, each marker sensor of the plurality of marker sensors may comprise a light source. In these and further examples, each marker sensor may comprise a polarized filter to block polarized light from some or all additional light sources disposed across the sample volume.

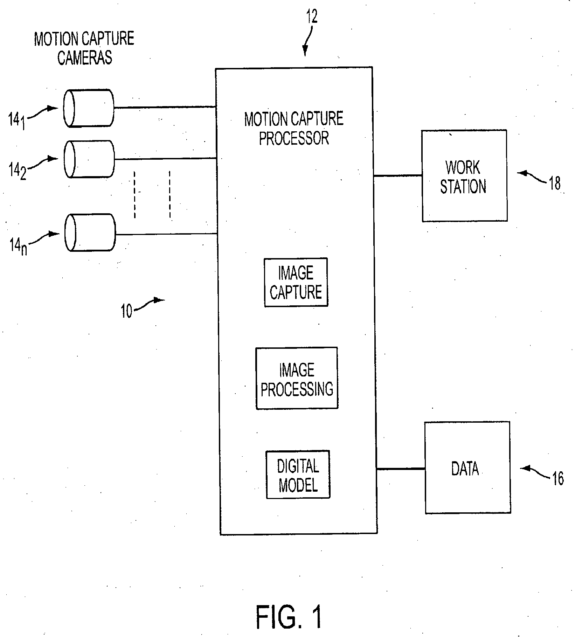

Referring first to **FIG. 1**, a block diagram illustrates an exemplary motion tracking system **10** in accordance with some embodiments. The motion tracking system **10** includes a motion capture processor **12** adapted to communicate with a plurality of marker sensors (*e.g*., cameras) **14₁-14ₙ.** The motion capture processor **12** may comprise a programmable computer having a data storage device **16** adapted to enable the storage of associated data files. A computer workstation **18** may be coupled to the motion capture processor **12** using a network to facilitate data acquisition, storage, and/or analysis. The marker sensors **14₁-14ₙ** may be arranged with respect to the sample volume to capture the position over time of fixed points on the surface of one or more plant samples growing within the sample volume.

At least one plant sample may be marked with markers that are detected by the marker sensors 1**4₁-14ₙ** during a period of time wherein the plant sample is situated within the sample volume. The markers may be, for example and without limitation, reflective elements (*e.g*., beads, tape, and paint), retroreflective elements, illuminated elements, LEDs, and radiotransmitter tags (*see* U.S. Patent 7,009,561). Alternatively, in other embodiments (for example, those utilizing markerless motion tracking), at least one plant sample that is not marked with markers may be situated within the sample volume during a period of sampling time. In particular examples, a plant sample may be marked with one or more markers (*e.g*., reflective beads) disposed at one or more position(s) on the plant sample, for example and without limitation, positions on the shoot system; root system (when the root system is growing in a medium through which a specific marker signal may pass, such as a translucent growth medium); shoot tip; apical bud; epidermis; flower; lateral bud; node; internode; leaf; leaf tip; apical meristems; lateral meristems; and ground tissue.

One marker, or increasing numbers of markers in excess of one marker, may be used to mark a single plant sample. For example and without limitation, 1, 2, 3, 4, 5, 6, 7, 8, 9, 10, about 20, about 30, about 40, about 50, or more markers may be used in certain embodiments. The use of increasing numbers of markers may provide a corresponding increase in resolution in a plant growth measurement that is ultimately obtained. Markers may have a width or diameter of, for example, at least about 2 millimeters (*e.g*., about 2 mm, about 4 mm, about 6 mm, about 8 mm, about 10 mm, about 15 mm, about 20 mm, about 25 mm, about 30 mm, about 40 mm, about 50 mm, about 75 mm, or more). The position of the centroid of one or more of the markers of any diameter may be calculated by methods known to those of skill in the art of image analysis.

The motion capture processor **12** processes two-dimensional images received from the marker sensors **14₁-14ₙ** to produce a three-dimensional digital representation of the captured motion. Particularly, the motion capture processor **12** may receive the two-dimensional data from each camera, and save the data in the form of multiple data files into a data storage device **16** as part of an image capture process. The two-dimensional data files may then be resolved into a single set of three-dimensional coordinates that are linked together in the form of trajectory files representing movement of individual markers as part of an image processing process. The image processing process uses images from one or more marker sensors to determine the location of each marker. For example, a marker may only be visible to a subset of the marker sensors due to occlusion by plant parts or other elements within the sample volume. In that case, the image processing function of the motion capture processor **12** uses images from other marker sensors that have an unobstructed view of that marker to determine the marker's location in space.

By using images from multiple cameras to determine the location of a marker, the image processing function evaluates the image information from multiple angles and uses a triangulation process to determine the spatial location. Kinetic calculations are then performed on the trajectory files to generate the digital representation reflecting, for example, extension or displacement of plant part(s) and/or plant tissues corresponding to growth of the plant. Using the spatial information over time, the calculations determine the progress of each marker as it moves through space. A suitable data management process may be used to control the storage and retrieval of the large number of files associated with the entire process to/from the data storage device **16**. The motion capture processor **12** or a linked workstation **18** may utilize a commercial software package (such as may be available from Vicon Motion Systems, Motion Analysis Corp., etc.) to perform these and other data processing functions.

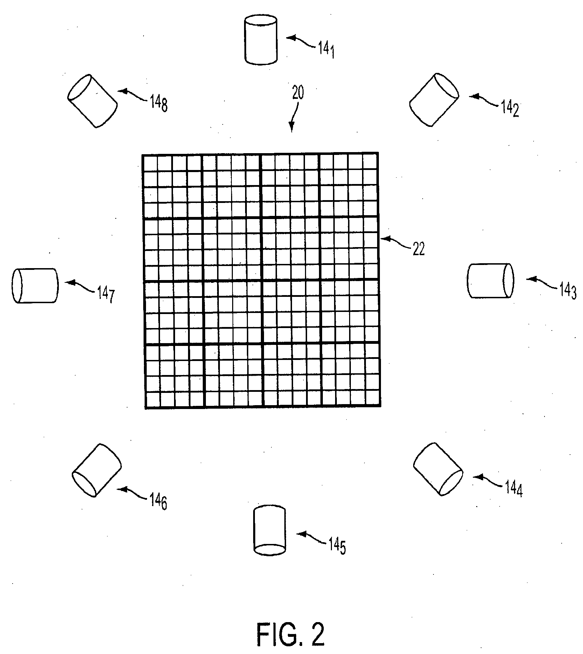

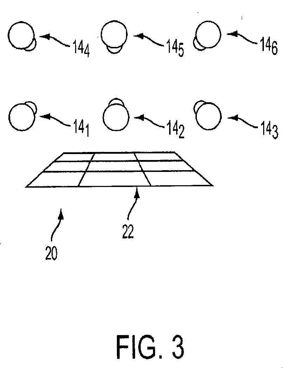

**FIGs. 2** and **3** illustrate an exemplary sample volume **20** according to some embodiments surrounded by a plurality of motion capture cameras. The sample volume **20** includes a peripheral edge **22**. The sample volume **20** is illustrated as a rectangular-shaped region subdivided by grid lines. It should be appreciated that the sample volume **20** actually comprises a three-dimensional space, with the grid defining a lowest point (*e.g*., a lowest cross-sectional surface) of the sample volume **20**. Motion would be captured within the three-dimensional space above the lowest point (or surface). In some embodiments, the sample volume **20** comprises a lowest surface of about 4 feet by about 4 feet, with a height of approximately 8 feet above the lowest surface. Other size and shape sample volumes may also be selected for use in particular applications in view of the requirements thereof (*e.g*., the size of plants to be grown in the sample volume, and the size of the marker displacement to be measured) according to the discretion of the practitioner.

**FIG. 2** illustrates a top view of the sample volume **20** according to some embodiments with the plurality of motion capture cameras arranged around the peripheral edge **22** in a generally circular array. However, other patterns (*e.g*., one or more linear or rectangular arrays) may also be used. Individual marker sensors are illustrated as triangles with the acute angle representing the direction of the lens of the camera, so it should be appreciated that the plurality of marker sensors are directed toward the sample volume **20** from a plurality of distinct directions. In particular embodiments, the plurality of marker sensors includes, for example and without limitation, at least three marker sensors (*e.g*., 3, 4, 5, 6, 7, 8, 9, 10, 11, 12, 13, 14, 15, or more cameras). In further embodiments, two marker sensors may be used with at least one fixed reference marker (*i.e.*, a marker that is not subject to displacement during the sample period) within the sample volume that is visible to both marker sensors.

**FIG. 3** illustrates a side view of the sample volume **20** according to particular embodiments, with the plurality of motion capture cameras arranged into roughly two tiers above the lowest surface of the sample volume. A lower tier includes a plurality of marker sensors **14₁-14ₙ** arranged along or outside of the peripheral edges **22** of the sample volume **20**. In some examples, each of the lower tier marker sensors **14₁-14₃** are aimed slightly upward so as to not include a marker sensor roughly opposite the sample volume **20** from being included within the field of view. The marker sensors may include a light source (*e.g*., an array of light emitting diodes) used to illuminate the sample volume **20**. It may be desirable to not have a marker sensor "see" the light source of another marker sensor, for example, since the light source of a first motion capture camera may appear to a second motion capture camera as a bright reflectance that will overwhelm data from the reflective markers. This problem may be circumvented or mitigated by the physical disposition of marker sensors opposite one another across the sample volume **20**, and/or by the use of polarized filters disposed in front of the marker sensors' lenses. An upper tier includes a plurality of marker sensors **14₄-14₆** arranged along or outside of the peripheral edges **22** of the sample volume **20.** In some examples, each of the upper tier marker sensors **14₄-14₆** are aimed slightly downward so as to not include a camera roughly opposite the sample volume **20** from being included within the field of view.

The marker sensors of a first tier of marker sensors may each have a wider field of view than those of a second tier of marker sensors, enabling each marker sensor in the first tier to include a greater amount of the sample volume **20** within its respective field of view. It should be appreciated that numerous alternative arrangements of the marker sensors can also be advantageously utilized in particular examples. For example, a greater or lesser number of separate tiers of marker sensors may be utilized, and the actual height of each marker sensor within an individual tier may be varied.

In some embodiments, the marker sensors record images of the markers from many different angles, so that substantially all of the lateral surfaces of the plant sample are exposed to at least one marker sensor at all times. More specifically, the arrangement of marker sensors may provide that substantially all of the lateral surfaces of the plant sample are exposed to at least three marker sensors at all times. By placing the marker sensors at multiple heights, irregular surfaces can be modeled as each marker on the growing plant sample moves within the motion capture field **20**.

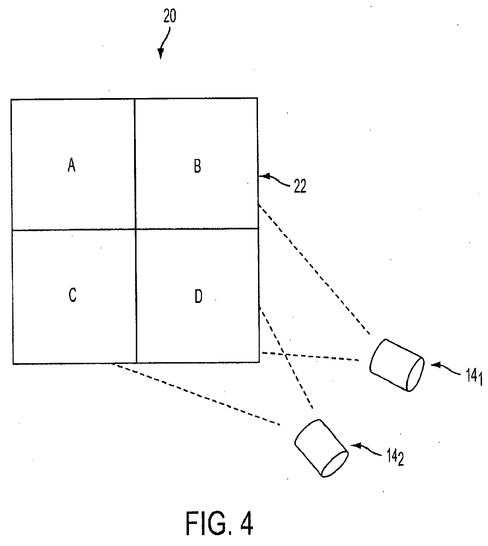

**FIG. 4** is a top view of the sample volume **20** according to some embodiments illustrating an exemplary arrangement of marker sensors (*e.g*., **14₁-14ₙ**). The sample volume **20** is graphically divided into quadrants, labeled A, B, C, and D. Other arrangements of marker sensors **14₁-14ₙ** may also be advantageously utilized in further examples. In the illustrated example, the marker sensors **14₁** and **14₂** are physically disposed adjacent to each other, yet offset horizontally from each other by a discemable distance. The marker sensors **14₁** and **14₂** are each focused on the front edge of quadrant D from an angle of approximately 45°. The first marker sensor **14₁** has a field of view that extends from partially into the front edge of quadrant B to the right end of the front edge of quadrant D. The second marker sensor **14₂** has a field of view that extends from the left end of the front edge of quadrant D to partially into the front edge of quadrant C. Thus, the respective fields of view of the first and second marker sensors **14₁** and **14₂** overlap over the substantial length of the front edge of quadrant D. A similar arrangement of marker sensors (*e.g*., **14₃-14ₙ**) may be included for each of the other outer edges (coincident with peripheral edge **22**) of quadrants A, B, C and D.

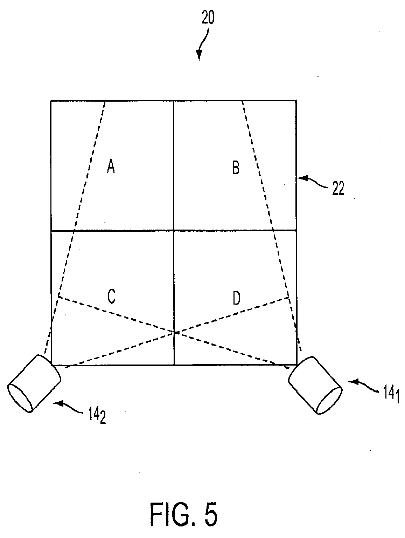

**FIG. 5** is a top view of the sample volume **20** illustrating another exemplary arrangement of marker sensors (e.g., **14₁-14ₙ**). As in **FIG. 4**, the sample volume **20** is graphically divided into quadrants A, B, C, and D. As in the embodiment of **FIG. 4**, the marker sensors **14₁-14ₙ** may be located at different heights. In the example illustrated in **FIG. 5**, the marker sensors **14₁** and **14₂** are located at corners of the sample volume **20** facing into the sample volume. These corner marker sensors **14₁** and **14₂** would record images that are not picked up by the other marker sensors (*e.g.*, **14₃-14ₙ**), such as due to occlusion. Other like marker sensors (*e.g.*, **14₃-14ₙ**) may also be located at the other comers of the sample volume **20**.

In some embodiments, the marker sensors all remain fixed in place, relative to the sample volume being imaged. This way, the motion capture processor **12** has a fixed reference point against which movement of the markers can be measured. In alternative embodiments, a portion of the marker sensors remain fixed, while others may be moved relative to the sample volume. The moveable cameras may be moved using computer-controlled servomotors, or may be moved manually. In these latter embodiments, the motion capture processor **12** may track the movement of the marker sensors, and remove this movement in the subsequent processing of the captured data to generate the three-dimensional digital representation of marker motion.

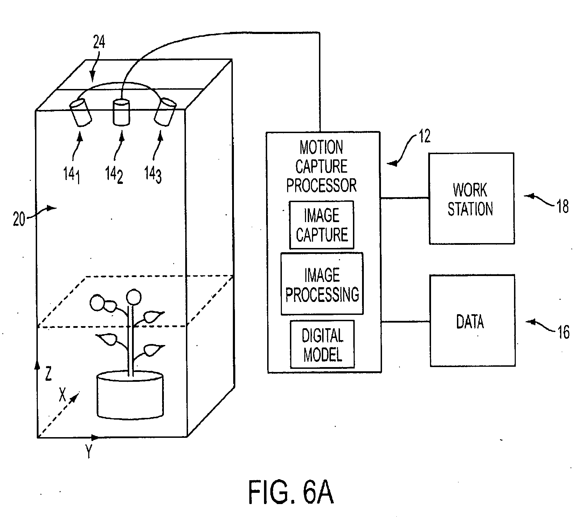

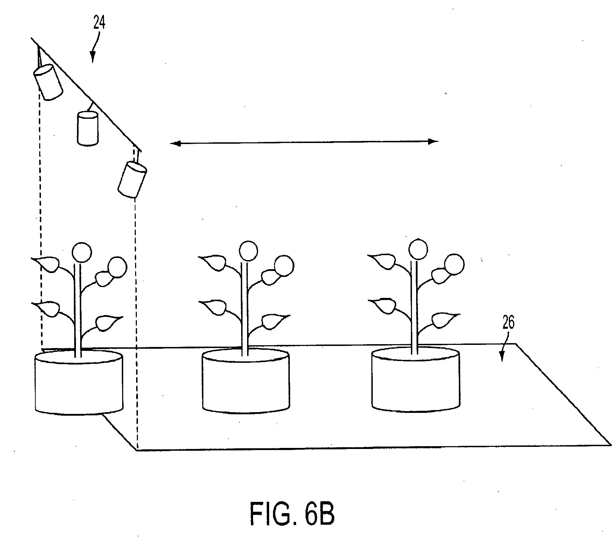

Some embodiments provide an automated apparatus and method for capturing plant growth kinetics data. Such embodiments may be adapted for high-throughput image data acquisition and analysis, and may thereby greatly decrease the cost and/or effort required for measurement of plant growth kinetics. In particular embodiments, the marker sensors may be moved (*e.g*., by fixed mounting on a moveable element), or particular plant samples may be moved. In some examples, however, the marker sensors and/or the plant samples in such automated systems remain fixed in relation to the sample volume. The principles involved in such automated systems are illustrated by way of example in **FIG. 6****.**

**FIG. 6a** shows an illustration of an exemplary embodiment including a camera array **14₁-14₃** connected to an overhead gantry **24** associated with a motion capture processor **12** (that may be capable of performing image capture, image processing, and assembly of a digital model), a linked workstation **18**, data storage device **16**, and equipment connections. **FIG. 6b** illustrates an embodiment including a high-throughput gravimetric automation platform. In this embodiment, the overhead gantry **24** may be moveable (*e.g*., in an automated manner) in at least one dimension, relative to the surface **26** holding the plant samples. In these and other embodiments, the surface **26** may be moveable (*e.g*., in an automated manner) in at least one dimension relative to the overhead gantry **24**.

It will be appreciated that an automated motion tracking system, as illustrated by the examples depicted in **FIG. 6**, limits the possible associated frame sizes and image sampling rates. For example, the time required to acquire one image for each sample in such an automated system will typically provide a lower limit on the image sampling rate for each particular sample. However, this is not necessarily the case, as multiple images may be acquired for one sample before the system adjusts to allow measurement of the next sample. Most plant growth processes occur with slow enough kinetics that an automated system can be designed to both accommodate a large number of samples, and acquire images with a frequency high enough to provide detailed spatiotemporal information regarding the analyzed process. The utilization of this feature of plant kinetics that is peculiar with regard to the sorts of movement typically sought to be captured in real-time is a particular feature of some embodiments of the present invention.

### VI. Screening plants having a growth trait of interest

In particular embodiments, a composition, method, and/or apparatus for capturing plant growth kinetics by motion tracking may be used to compare the growth of individual plants under the same or different environmental conditions, thereby providing a relatively inexpensive, rapid, and/or high-throughput system for screening plants for specific agronomic traits. Thus, some embodiments include methods of screening plants for a trait or phenotype that has an effect on the growth of a plant. Such traits and phenotypes include, for example and without limitation, drought tolerance, NUE, heat tolerance, and salt tolerance. Many desirable plant phenotypes involve changes in plant growth and development beyond what is exhibited in wild-type plants or other cultivars.

Live imaging is a first step in the measurement and modeling of live plant growth and development. Spatiotemporal mathematical models allow testing of plant growth hypotheses through dynamic simulations of growth. And finally, plant growth models and their predictions may be validated by further live imaging experiments. Accordingly, in some embodiments, motion tracking of plant growth kinetics is used to measure a kinetic parameter of plant growth in a plurality of plant samples. In these and further embodiments, mathematical models may be constructed to describe plant growth process(es), for example, to extrapolate the effects of such processes to different plants of the same or a related species.

Plant traits and phenotypes may be introduced into a plant, for example, through conventional plant breeding or genetic transformation. Both of these methodologies typically produce a large number of candidate plants (breeding progeny and putative transformants, respectively) that must be screened for occurrence of the trait or phenotype. Even screening for traits or phenotypes that are visible by simple inspection may be time-consuming and costly. Plant growth phenotypes may only be visible by conventional techniques after long periods of time, after which the effect of the phenotype on overall plant morphology may become apparent.

By using compositions, apparati, and methods of some embodiments of the present invention, plant growth phenotypes that occur too slowly to be observable by the naked human eye, but which also are occurring before any ultimate effects on plant morphology may be apparent, may be measured and modeled in real-time, with minimal manipulation of the plant sample during the data acquisition period. For example, by using an automated motion tracking system according to some embodiments, a large number of plant samples may be analyzed in a single platform in a high-throughput manner.

All publications and patents cited herein are hereby incorporated in their entirety.

The following examples are provided to illustrate certain particular features and/or embodiments, such as those described above. The examples should not be construed to limit the disclosure to the particular features or embodiments exemplified.

### EXAMPLES

### Example 1: Materials and Methods

### Plant Material Growth and Care

*Zea mays* c.v. B104 inbred plantlets, grown under optimal watering and nutrient conditions, were used to track plant kinetic measurements. Plantlets were grown in white PVC sewer drain pots (United Pipe Supply, Boise, ID), with the dimensions of 15" tall by 6" wide, in top soil (Tualatin Valley Landscape Supply, Tualatin, OR) blended with HP Promix (Growers Nursery Supply, Salem, OR) in a 1:1 ratio, and fertilized with Osmocote™ Plus 15-9-12 NPK, 3-4 month slow release fertilizer (Scott's Company, Marysville, OH). Seed was sown 1.5" deep, and soil was kept saturated with regular clear water irrigation events occurring every 3 days from sowing. Upon germination, plantlets were watered as needed to maintain saturated soil conditions.

Plantlets were grown under standard greenhouse conditions. Conditions included approximately 50% average incident light, 50% average relative humidity, and a diurnal temperature cycle between 28 °C and 35 °C, with a 16-hour daylight cycle. The light source consisted of an arrangement of alternating halogen and high sodium pressure light bulbs set for a minimum of 350 PAR at 1 meter above the bench that the plants were placed upon. At approximately the V4 stage of development, plantlets were used for imaging experiments, as described below.

### Imaging Station Description and Equipment Set-up

Digital Imaging. Positional changes of reflective markers placed on individual plant leaves were tracked, thereby monitoring plant kinetic measurements. The plant kinetic measurements recorded and monitored plant growth characteristics, such as leaf motion and elongation. The experiment was conducted using digital camera imaging of the reflective markers placed on plant leaves to track 2-dimensional changes in position. Image capture occurred with the use of a manually-maneuverable imaging gantry aluminum frame structure fitted with a mounting block capable of horizontal motion. This structure was able to accommodate mounting of a standard digital camera pointed at a 90° angle to the plant canopy plane. The camera flash option was turned off during imaging to prevent excess illumination of shiny leaf surfaces, and thus avoid marker visibility occlusion during image processing. Two perpendicular meter sticks were mounted in plane with the plant canopy to assist with calibration of image scale for digital image processing.

OPTITRACK™ Motion Tracking Image Capture. For motion tracking experiments, three OPTITRACK™ FLEX:V100R2 cameras (Natural Point, Corvalis, OR) were mounted on the ceiling (approximately 8' from ground level) of an imaging station (8'H x 4'W x 4'L). The interior of the imaging station was painted with a black matte finish throughout, and the front entry was fixed with a black flexible tarp to prevent illumination or reflectance from outside light sources or shiny surfaces. The cameras were placed equidistant from each other with each camera angled to face the center floor of the imaging booth, resulting in a capture volume of approximately 3 m³ with minimum camera coverage of 2 m³. The cameras were connected to each other using the included USB OPTIHUB™, which was then connected to a PANASONIC TOUGHBOOK™ (Panasonic, Kadoma, Osaka, Japan) laptop computer loaded with the OPTITRACK™ Tracking Tool software for data/sequence file capture. Capture rate was set at approximately 1 frame per second, with recordings generally lasting no longer than 5-10 seconds per imaging event. Camera exposure level was set at 55 lumens, threshold at 160 (no units), and intensity at 15 ms. A schematic of this setup is illustrated in **FIG. 6**. Calibration was performed prior to every measurement event using the OPTITRACK™ calibration wand for 10 seconds to define and capture as many unique points within the measured volume, and to ensure that the boundaries of the measurement volume were defined. Surface level was identified using the OPTITRACK™ reference square tool. The cameras and software were actively in operation only during the measurement period. At other times, the equipment was powered down.

Digital Imaging Proof of Concept Assay. The kinetic changes in leaf elongation and general motion of *Zea mays* plants were monitored and measured by using reflective markers and digital imaging. The kinetic changes in leaf elongation and general motion in a subset of plants grown under standard, well-watered conditions were compared to plants grown under acutely drought-stressed conditions (described below). At the V6 developmental stage, 3 mm reflective markers were placed on either side of the newest emerging leaf (typically leaf 11-12), approximately 0.5 cm from the tip of the leaf. Plants were placed under the manual imaging gantry, and left to grow and develop under standard greenhouse conditions. Imaging began with all plantlets maintained under well-watered conditions. Water was then withheld from plantlets in the drought block, and over the next 6 days images were taken in darkness between 5:00 a.m. and 6:15 a.m. pacific standard time (PST) to minimize outside light sources interfering with marker detection (plants in this preliminary study were kept on bench tops and not placed in a darkened imaging station).

Digital Image Processing. Imaging ceased when the plantlets grown in drought conditions had completely rolled and wilted. Digital images were processed by manually drawing gridlines of a set metric calibrated to the meter stick visible in each image. For preliminary assessment, images were marked using graphics software, and changes were visually assessed across frames.

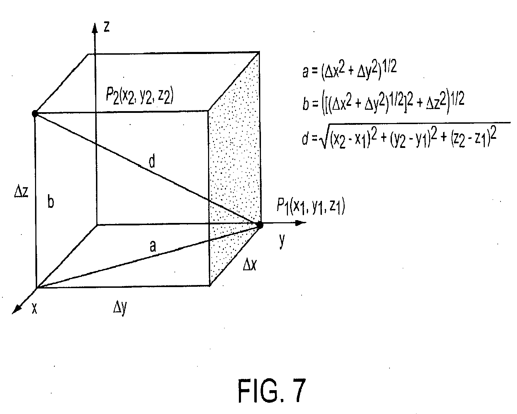

Image Sequence Processing. After capture of the image sequence, data was converted to .csv format for processing in Microsoft EXCEL™ (Microsoft, Seattle, WA). Each marker detected during the measurement period was given a specific three-dimensional coordinate (x, y, z) for each frame. Differences across time points between these coordinate points were calculated for each marker of each plantlet and leaf using a standard three-dimensional distance formula. **FIG. 7****.**

### Example 2: OPTITRACK™ Motion Tracking Imaging Assay

Three consecutive designs were run to validate the motion tracking utility in greenhouse measurements, each with distinct objectives of increasing complexity.

### Single plant, single leaf per plant tracked

The first design measured displacement of markers on a single leaf of an individual plantlet in three-dimensional space. Plant kinetic measurements began at the V4 developmental stage. Two reflective tracking markers (4 mm in length) were placed on either side of the tip of the newest emerging leaf (typically leaf number 8 or 9), with at least 1 cm of leaf tip protruding from the primary whorl. Measurements were taken between 6:00 a.m. and 12:00 noon PST, for a period of 7 days. The plantlet was placed on a low platform rolling utility cart, and each day the cart was moved to the center of the imaging station, and pushed against positioning blocks to ensure identical positioning with each measurement event. With an imaging station entry tarp completely closed, data capture proceeded for approximately 10 seconds. Immediately following data capture, the plantlet was placed back into the standard greenhouse growth environment.

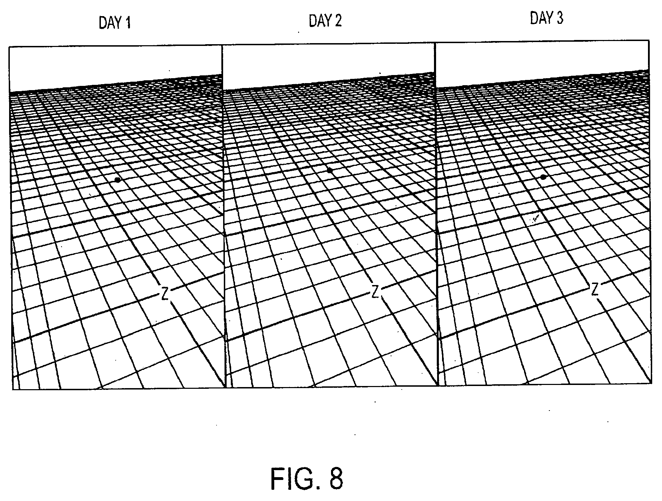

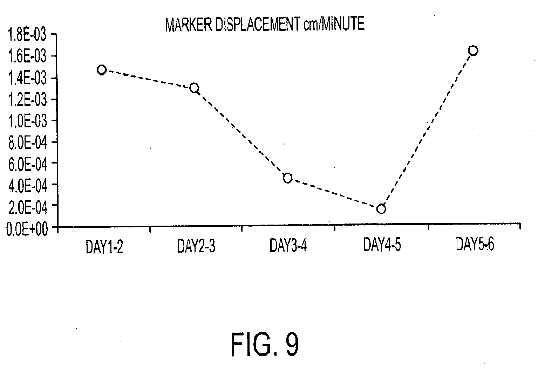

Plant leaf motion and quantifiable measurement of displacement over time was captured using the cameras and tracking tool software. **FIG. 8** illustrates the screenshot pictures which were monitored with the tracking tool. **FIG. 9** illustrates the daily change in three-dimensional position in space from the initial location to the final location, measured in meters (converted to cm).

### Motion tracking of three separate leaves on a single plant in three-dimensional space

Next, displacement was measured in three-dimensional space of markers attached to three separate leaves of increasing maturity of an individual plantlet. Plant kinetics measurements began at the V4 developmental stage. The first set of tracking markers was placed (using similar techniques as the first experiment) on either side of the tip of the newest emerging leaf, with at least 1 cm of leaf tip protruding from the primary whorl (typically leaf 8-9). The second set was placed 1 cm from the end of the tip of the leaf on the next newest leaf (typically leaf 7-8), and a third set was placed on the next newest leaf (typically leaf 6-7). Measurements were taken between 6:00 a.m. and 12:00 noon PST, for a period of 7 days. As described previously, the plantlets were placed on the rolling utility cart and measured in the darkened imaging station for 10 seconds each day, after which the plantlets were returned back to the standard growth environment.

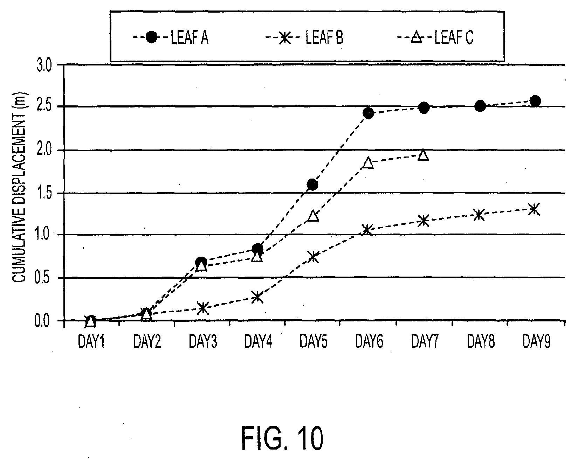

Detection and quantifiable measurement of the displacement of markers placed on the tips of three consecutively newer emerging leaves of a single plant was found to be possible using this motion tracking system. **FIG. 10** illustrates the cumulative displacement of markers attached to three different leaves over time.

### Multiple plants, single leaf per plant

Next, displacement was measured in three-dimensional space of markers attached to a single leaf on a row of 9 separate plantlets. Initiation of measurements began at the V4 developmental stage. Markers were placed on leaves (using similar techniques as the first experiment), typically on leaf 8-9 of each plantlet. Measurements were taken between 6:00 a.m. and 12:00 noon PST, for a period of 7 days. The plantlets were aligned in a single row on the utility cart, without gaps between pot edges, and measurements were taken once positioned in the darkened imaging station for 10 seconds each day, after which plantlets were returned to the standard growth environment.

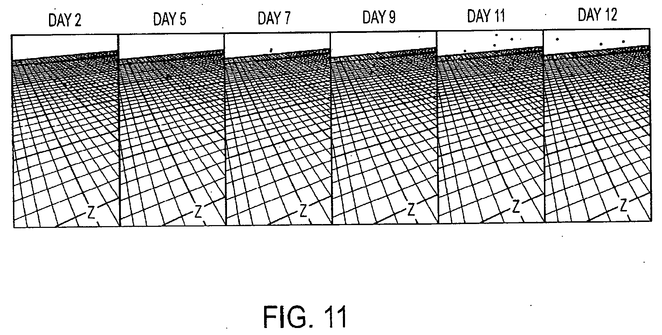

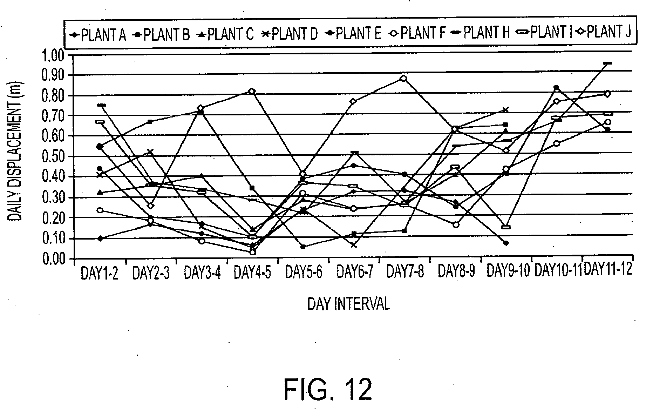

We found that tracking reflective markers on multiple plants in three-dimensional space was possible. **FIG. 11** illustrates screenshot pictures of tracking tools image capture over the course of 12 days. **FIG. 12** illustrates the daily leaf displacement values for multiple plants measured using the motion tracking tools.

### Example 3: Digital Tracking Distinguishes Visual Differences in Leaf Elongation Between Well-Watered and Water-Stressed Plants

Z. *mays* plants that were either drought-stressed or maintained under optimal watering conditions were monitored for reduced motion and leaf elongation. Results from the experiments using digital photography and highly-reflective markers placed on a single leaf showed qualitative differences in plant leaf elongation over time between the two treatment groups. The drought-stressed plants initially demonstrated growth patterns similar to the well-watered controls. However, by 4-5 days of acute drought conditions, the drought-stressed plants exhibited visually reduced motion and displacement of the markers on the leaf surface, ultimately leading to completely static positioning of the markers in relation to the background. By day 6, it appeared that growth had ceased in the drought stressed plants, with leaves rolled and curled, whereas well-watered plants continued to exhibit leaf elongation that extended past the field of view.

This study was also conducted to determine the logistics of using reflective markers, optimal positioning, and testing of the visibility of markers in a canopy of multiple plants to identify possible obstruction issues related to crowded plant canopies. Digital images depicting daily changes in select reflective markers of well-watered and stressed plants were tracked. We found that while some markers did temporarily disappear from view, most of the markers remained visible over the course of the experiment.

## Claims

1. A plant sample growth motion tracking and imaging station, comprising:
a sample volume (20) adapted to contain at least one plant sample having at least one marker defining physical points on the plant sample;
a plurality of marker sensors (14₁-14ₙ) arranged at the periphery of the sample volume; and
a motion capture processor (12) coupled to the plurality of marker sensors to receive motion capture data from the plurality of marker sensors, and produce a digital representation reflecting growth of the plant sample.

2. The plant sample growth motion tracking and imaging station of claim 1, wherein the digital representation comprises spatial information in three dimensions and temporal information.

3. The plant sample growth motion tracking and imaging station of claim 2, wherein at least one of the marker sensors are cameras.

4. The plant sample growth motion tracking and imaging station of claim 3, wherein the sample volume is adapted to contain at least one plant sample having at least one marker comprising a reflective element.

5. The plant sample growth motion tracking and imaging station of claim 4, wherein the at least one marker comprising a reflective element is a bead.

6. The plant sample growth motion tracking and imaging station of claim 4, wherein each of the at least one marker has a width or diameter of between about 2 and about 10 millimeters.

7. The plant sample growth motion tracking and imaging station of claim 4, wherein the sample volume is adapted to contain at least one plant sample having 2 or more markers.

8. The plant sample growth motion tracking and imaging station of claim 2, wherein the sample volume is adapted to contain at least two plants.

9. The plant sample growth motion tracking and imaging station of claim 1, wherein the motion capture processor is capable of performing image capture and image processing.

10. The plant sample growth motion tracking and imaging station of claim 1, further comprising a workstation (18) and a data storage device (16), wherein the workstation and data storage device are coupled to the motion capture processor.

11. The plant sample growth motion tracking and imaging station of claim 2, further comprising a moveable overhead gantry (24) comprising at least one of the plurality of marker sensors.

12. The plant sample growth motion tracking and imaging station of claim 11, wherein the moveable overhead gantry comprises all of the plurality of marker sensors.

13. A method for monitoring the growth kinetics of a plant sample, the method comprising:
defining a sample volume (20) adapted to contain at least one plant sample having at least one marker defining physical points on the plant sample;
arranging a plurality of marker sensors (14₁-14ₙ) at the periphery of the sample volume;
acquiring motion capture data utilizing the plurality of marker sensors; and
receiving motion capture data from the plurality of marker sensors and producing a digital representation reflecting growth of the plant sample.

14. The method according to claim 13, wherein the digital representation comprises spatial information in three dimensions and temporal information.

15. The method according to claim 14, wherein at least one of the marker sensors are cameras.

16. The method according to claim 13, further comprising:
introducing into the sample volume a plant sample having two or more markers defining physical points on the plant sample.

17. The method according to claim 13, further comprising:
introducing into the sample volume at least two plant samples having at least one marker defining physical points on the plant sample.

18. The method according to claim 13, wherein at least one of the plurality of marker sensors are attached to a means for moving the sample volume without moving the marker sensors in relation to the sample volume.

19. The method according to claim 18, wherein the means for moving the sample volume without moving the marker sensors in relation to the sample volume is a moveable overhead gantry (24), wherein the marker sensors are attached to the gantry.

20. The method according to claim 18, wherein the means for moving the sample volume without moving the marker sensors in relation to the sample volume is controlled by an automated system.

21. A method for screening a plant sample for a growth trait of interest, the method comprising:
defining a sample volume (20) adapted to contain at least one plant sample having at least one marker defining physical points on the plant sample;
arranging a plurality of marker sensors (14₁-14ₙ) at the periphery of the sample volume;
introducing into the sample volume a plant sample having at least one marker defining physical points on the plant sample;
acquiring motion capture data utilizing the plurality of marker sensors;
receiving motion capture data from the plurality of marker sensors and producing a digital representation reflecting growth of the plant sample; and
analyzing the digital reflection to determine whether the plant sample comprises the growth trait of interest.

22. The method according to claim 21, wherein the plant sample is a whole plant.

23. The method according to claim 21, wherein the growth trait of interest is a trait providing increased plant growth in a stress condition.

24. The method according to claim 23, wherein the stress condition is selected from a group comprising drought, low light, high salt, low nitrogen, chemical exposure, and pest infestation.

## Patentansprüche

1. Eine Station zur Nachverfolgung und Abbildung der Wachstumsbewegung einer Pflanzenprobe umfassend:
einen dreidimensionalen Probenraum (20), der geeignet ist, mindestens eine Pflanzenprobe mit mindestens einer Markierung, die physische Punkte auf der Pflanzenprobe definiert, zu enthalten;
eine Mehrzahl von Markierungssensoren (14₁-14ₙ), die am Rand des dreidimensionalen Probenraums angeordnet sind und
einen Bewegungsaufnahmeprozessor (12), der mit der Mehrzahl der Markierungssensoren verbunden ist, um Bewegungsaufnahmedaten von der Mehrzahl der Markierungssensoren zu empfangen und eine digitale Darstellung zu erzeugen, die das Wachstum der Pflanzenprobe wiedergibt.

2. Die Station zur Nachverfolgung und Abbildung der Wachstumsbewegung einer Pflanzenprobe gemäß Anspruch 1, wobei die digitale Darstellung räumliche Informationen in drei Dimensionen und zeitliche Informationen umfasst.

3. Die Station zur Nachverfolgung und Abbildung der Wachstumsbewegung einer Pflanzenprobe gemäß Anspruch 2, wobei mindestens einer der Markierungssensoren eine Kamera ist.

4. Die Station zur Nachverfolgung und Abbildung der Wachstumsbewegung einer Pflanzenprobe gemäß Anspruch 3, wobei der dreidimensionale Probenraum geeignet ist, mindestens eine Pflanzenprobe mit mindestens einer Markierung, die ein reflektierendes Element umfasst, zu enthalten.

5. Die Station zur Nachverfolgung und Abbildung der Wachstumsbewegung einer Pflanzenprobe gemäß Anspruch 4, wobei die mindestens eine Markierung, die ein reflektierendes Element umfasst, ein Kügelchen ist.

6. Die Station zur Nachverfolgung und Abbildung der Wachstumsbewegung einer Pflanzenprobe gemäß Anspruch 4, wobei jede der mindestens einen Markierung eine Breite oder einen Durchmesser von zwischen etwa 2 und etwa 10 Millimetern aufweist.

7. Die Station zur Nachverfolgung und Abbildung der Wachstumsbewegung einer Pflanzenprobe gemäß Anspruch 4, wobei der dreidimensionale Probenraum geeignet ist, mindestens eine Pflanzenprobe mit 2 oder mehr Markierungen zu enthalten.

8. Die Station zur Nachverfolgung und Abbildung der Wachstumsbewegung einer Pflanzenprobe gemäß Anspruch 2, wobei der dreidimensionale Probenraum geeignet ist, mindestens zwei Pflanzen zu enthalten.

9. Die Station zur Nachverfolgung und Abbildung der Wachstumsbewegung einer Pflanzenprobe gemäß Anspruch 1, wobei der Bewegungsaufnahmeprozessor in der Lage ist, Bildaufzeichnung und Bildbearbeitung durchzuführen.

10. Die Station zur Nachverfolgung und Abbildung der Wachstumsbewegung einer Pflanzenprobe gemäß Anspruch 1, weiterhin umfassend eine Workstation (18) und eine Datenspeichervorrichtung (16), wobei die Workstation und die Datenspeichervorrichtung mit dem Bewegungsaufnahmeprozessor verbunden sind.

11. Die Station zur Nachverfolgung und Abbildung der Wachstumsbewegung einer Pflanzenprobe gemäß Anspruch 2, weiterhin umfassend eine bewegliche Brücke, die mindestens einen der Mehrzahl der Markierungssensoren umfasst.

12. Die Station zur Nachverfolgung und Abbildung der Wachstumsbewegung einer Pflanzenprobe gemäß Anspruch 11, wobei die bewegliche Brücke alle der Mehrzahl der Markierungssensoren umfasst.

13. Ein Verfahren zur Beobachtung der Wachstumskinetik einer Pflanzenprobe, wobei das Verfahren umfasst:
Definieren eines dreidimensionalen Probenraums (20), der geeignet ist, mindestens eine Pflanzenprobe mit mindestens einer Markierung, die physische Punkte auf der Pflanzenprobe definiert, zu enthalten;
Anordnen einer Mehrzahl von Markierungssensoren (14₁-14ₙ) am Rand des dreidimensionalen Probenraums;
Erfassen von Bewegungsaufnahmedaten unter Verwendung der Mehrzahl von Markierungssensoren und
Empfangen der Bewegungsaufnahmedaten von der Mehrzahl der Markierungssensoren und Erstellen einer digitalen Darstellung, die das Wachstum der Pflanzenprobe wiedergibt.

14. Das Verfahren gemäß Anspruch 13, wobei die digitale Darstellung räumliche Informationen in drei Dimensionen und zeitliche Informationen umfasst.

15. Das Verfahren gemäß Anspruch 14, wobei mindestens einer der Markierungssensoren eine Kamera ist.

16. Das Verfahren gemäß Anspruch 13, weiterhin umfassend das Einbringen einer Pflanzenprobe mit zwei oder mehr Markierungen, die physische Punkte auf der Pflanzenprobe definieren, in den dreidimensionalen Probenraum.

17. Das Verfahren gemäß Anspruch 13, weiterhin umfassend das Einführen von mindestens zwei Pflanzenproben mit mindestens einer Markierung, die physische Punkte auf der Pflanzenprobe definiert, in den dreidimensionalen Probenraum.

18. Das Verfahren gemäß Anspruch 13, wobei mindestens einer der Mehrzahl von Markierungssensoren an einer Vorrichtung zur Bewegung des dreidimensionalen Probenraums ohne Bewegung der Markierungssensoren im Verhältnis zu dem dreidimensionalen Probenraum angebracht ist.

19. Das Verfahren gemäß Anspruch 18, wobei die Vorrichtung zur Bewegung des dreidimensionalen Probenraums ohne Bewegung der Markierungssensoren im Verhältnis zu dem dreidimensionalen Probenraum eine bewegliche Brücke (24) ist, wobei die Markierungssensoren an der Brücke angebracht sind.

20. Das Verfahren gemäß Anspruch 18, wobei die Vorrichtung zur Bewegung des dreidimensionalen Probenraums ohne Bewegung der Markierungssensoren im Verhältnis zu dem dreidimensionalen Probenraum durch ein automatisiertes System geregelt wird.

21. Ein Verfahren zum Screenen einer Pflanzenprobe nach einem Wachstumsmerkmal von Interesse, wobei das Verfahren umfasst:
Definieren eines dreidimensionalen Probenraums (20), der geeignet ist, mindestens eine Pflanzenprobe mit mindestens einer Markierung, die physische Punkte auf der Pflanzenprobe definiert, zur enthalten;
Anordnen einer Mehrzahl von Markierungssensoren (14₁-14ₙ) am Rand des dreidimensionalen Probenraums;
Einführen einer Pflanzenprobe mit mindestens einer Markierung, die physische Punkte auf der Pflanzenprobe definiert, in den dreidimensionalen Probenraum;
Erfassen von Bewegungsaufnahmedaten unter Verwendung der Mehrzahl von Markierungssensoren;
Empfangen von Bewegungsaufnahmedaten von der Mehrzahl der Markierungssensoren und Erstellen einer digitalen Darstellung, die das Wachstum der Pflanzenprobe wiedergibt und
Analysieren der digitalen Wiedergabe, um zu bestimmen, ob die Pflanzenprobe das Wachstumsmerkmal von Interesse umfasst.

22. Das Verfahren gemäß Anspruch 21, wobei die Pflanzenprobe eine ganze Pflanze ist.

23. Das Verfahren gemäß Anspruch 21, wobei das Wachstumsmerkmal von Interesse ein Merkmal ist, welches unter einer Stressbedingung ein erhöhtes Pflanzenwachstum vermittelt.

24. Das Verfahren gemäß Anspruch 23, wobei die Stressbedingung aus der Gruppe umfassend Dürre, wenig Licht, hoher Salzgehalt, niedriger Stickstoffgehalt, Kontakt mit chemischen Substanzen und Schädlingsbefall auswählt ist.

## Revendications

1. Station d'imagerie et de suivi du mouvement de croissance d'un échantillon de végétal, comportant :
- un volume à échantillon (20), adapté pour contenir au moins un échantillon de végétal portant au moins un marqueur qui définit des points physiques sur l'échantillon de végétal,
- un ensemble de plusieurs capteurs (14₁ - 14ₙ) de marqueur, disposés à la périphérie du volume à échantillon,
- et un processeur (12) de capture de mouvement, couplé aux capteurs de marqueur pour recevoir des données de capture de mouvement provenant des capteurs de marqueur et produire une représentation numérique reflétant la croissance de l'échantillon de végétal.

2. Station d'imagerie et de suivi du mouvement de croissance d'un échantillon de végétal, conforme à la revendication 1, dans laquelle la représentation numérique comprend des informations spatiales en trois dimensions et des informations temporelles.

3. Station d'imagerie et de suivi du mouvement de croissance d'un échantillon de végétal, conforme à la revendication 2, dans laquelle au moins l'un des capteurs de marqueur est une caméra.

4. Station d'imagerie et de suivi du mouvement de croissance d'un échantillon de végétal, conforme à la revendication 3, dans laquelle le volume à échantillon est adapté pour contenir au moins un échantillon de végétal portant au moins un marqueur qui comporte un élément réfléchissant.

5. Station d'imagerie et de suivi du mouvement de croissance d'un échantillon de végétal, conforme à la revendication 4, dans laquelle le marqueur, au nombre d'au moins un, qui comporte un élément réfléchissant est une perle.

6. Station d'imagerie et de suivi du mouvement de croissance d'un échantillon de végétal, conforme à la revendication 4, dans laquelle chacun des marqueurs au nombre d'au moins un présente une largeur ou un diamètre qui vaut entre environ 2 et environ 10 millimètres.

7. Station d'imagerie et de suivi du mouvement de croissance d'un échantillon de végétal, conforme à la revendication 4, dans laquelle le volume à échantillon est adapté pour contenir au moins un échantillon de végétal portant deux marqueurs ou plus.

8. Station d'imagerie et de suivi du mouvement de croissance d'un échantillon de végétal, conforme à la revendication 2, dans laquelle le volume à échantillon est adapté pour contenir au moins deux végétaux.

9. Station d'imagerie et de suivi du mouvement de croissance d'un échantillon de végétal, conforme à la revendication 1, dans laquelle le processeur de capture de mouvement est capable d'effectuer une capture d'image et un traitement d'image.

10. Station d'imagerie et de suivi du mouvement de croissance d'un échantillon de végétal, conforme à la revendication 1, qui comprend en outre une station de travail (18) et un dispositif (16) de stockage de données, et dans laquelle la station de travail et le dispositif de stockage de données sont couplés au processeur de capture de mouvement.

11. Station d'imagerie et de suivi du mouvement de croissance d'un échantillon de végétal, conforme à la revendication 2, qui comprend en outre un portique supérieur mobile portant au moins l'un des plusieurs capteurs de marqueur.

12. Station d'imagerie et de suivi du mouvement de croissance d'un échantillon de végétal, conforme à la revendication 11, dans laquelle le portique supérieur mobile porte tout l'ensemble des plusieurs capteurs de marqueur.

13. Procédé de suivi de la cinétique de croissance d'un échantillon de végétal, lequel procédé comporte les étapes suivantes :
- définir un volume à échantillon (20), adapté pour contenir au moins un échantillon de végétal portant au moins un marqueur qui définit des points physiques sur l'échantillon de végétal,
- disposer un ensemble de plusieurs capteurs (14₁ - 14ₙ) de marqueur à la périphérie du volume à échantillon,
- acquérir des données de capture de mouvement au moyen de ces plusieurs capteurs de marqueur,
- et recevoir des données de capture de mouvement provenant de ces plusieurs capteurs de marqueur et produire une représentation numérique reflétant la croissance de l'échantillon de végétal.

14. Procédé conforme à la revendication 13, dans lequel la représentation numérique comprend des informations spatiales en trois dimensions et des informations temporelles.

15. Procédé conforme à la revendication 14, dans lequel au moins l'un des capteurs de marqueur est une caméra.

16. Procédé conforme à la revendication 13, qui comprend en outre le fait d'introduire dans le volume à échantillon un échantillon de végétal portant deux marqueurs ou plus qui définissent des points physiques sur l'échantillon de végétal.

17. Procédé conforme à la revendication 13, qui comprend en outre le fait d'introduire dans le volume à échantillon au moins deux échantillons de végétal portant au moins un marqueur qui définit des points physiques sur l'échantillon de végétal.

18. Procédé conforme à la revendication 13, dans lequel au moins l'un des capteurs de marqueur est attaché à un moyen qui permet de déplacer le volume à échantillon sans déplacer les capteurs de marqueur par rapport au volume à échantillon.

19. Procédé conforme à la revendication 18, dans lequel le moyen qui permet de déplacer le volume à échantillon sans déplacer les capteurs de marqueur par rapport au volume à échantillon est un portique supérieur mobile, et dans lequel les capteurs de marqueur sont attachés à ce portique.

20. Procédé conforme à la revendication 18, dans lequel le moyen qui permet de déplacer le volume à échantillon sans déplacer les capteurs de marqueur par rapport au volume à échantillon est commandé par un système automatisé.

21. Procédé de recherche par criblage d'une caractéristique de croissance intéressante chez un échantillon de végétal, lequel procédé comporte les étapes suivantes :
- définir un volume à échantillon (20), adapté pour contenir au moins un échantillon de végétal portant au moins un marqueur qui définit des points physiques sur l'échantillon de végétal,
- disposer un ensemble de plusieurs capteurs (14₁ - 14ₙ) de marqueur à la périphérie du volume à échantillon,
- introduire dans le volume à échantillon un échantillon de végétal portant au moins un marqueur qui définit des points physiques sur l'échantillon de végétal,
- acquérir des données de capture de mouvement au moyen desdits plusieurs capteurs de marqueur,
- recevoir les données de capture de mouvement provenant de ces plusieurs capteurs de marqueur et produire une représentation numérique reflétant la croissance de l'échantillon de végétal,
- et analyser ce reflet numérique pour déterminer si l'échantillon de végétal porte la caractéristique de croissance intéressante.

22. Procédé conforme à la revendication 21, dans lequel l'échantillon de végétal est un plant entier.

23. Procédé conforme à la revendication 21, dans lequel la caractéristique de croissance intéressante est une caractéristique qui assure une croissance accrue du végétal quand celui-ci est soumis à une cause de stress.

24. Procédé conforme à la revendication 23, dans lequel la cause de stress est choisie dans l'ensemble formé par les conditions suivantes : sécheresse, faible luminosité, forte salinité, faible apport d'azote, exposition à un produit chimique, et infestation par un nuisible.
